# EUROPÄISCHE PATENTSCHRIFT

(11) **EP 0 818 441 B1**
(45) Veröffentlichungstag und Bekanntmachung des Hinweises auf die Patenterteilung: **02.05.2002**
(21) Anmeldenummer: 97110632.3
(22) Anmeldetag: 30.06.1997
(51) Int. Cl.: C07C 269/08

(54) **Verfahren zur destillativen Reinigung von Diurethanen**
Process for performing distillative purification of diurethanes
Procédé de purification distillative de diuréthanes

(30) Priorität: 11.07.1996 DE 19627906
(43) Veröffentlichungstag der Anmeldung: 14.01.1998
(73) Patentinhaber: BAYER AG, 51368 Leverkusen (DE)
(72) Erfinder: Kaltenbacher, Erwin Dr., 47800 Krefeld (DE); Mendoza-Frohn, Christine Dr., 40699 Erkrath (DE); Penninger, Stefan Dr., 50259 Pulheim (DE); Wershofen, Stefan Dr., Houston, TX, 77059 (US)

(56) Entgegenhaltungen:
- EP-A- 0 547 444
- EP-A- 0 749 959

## Beschreibung

Die vorliegende Erfindung betrifft ein Verfahren zur destillativen Reinigung von organischen Diurethanen durch Trägergasdestillation.

Die phosgenfreie Herstellung von Diisocyanaten, die technisch als Ausgangsprodukte zur Herstellung von Polyurethankunststoffen verwendet werden, erfolgt überwiegend durch thermische Spaltung von Diurethanen, die ihrerseits aus Diaminen oder Dinitroverbindungen zugänglich sind (siehe z.B. DE-A 3 108 990, 3 146 627, 3 215 591, 3 339 300, 3 915 181 und 3 915 183, EP-A 0 555 628, JP 59106448 (1984)). Entscheidend für das Gelingen der thermischen Urethanspaltung ist jedoch der Reinheitsgrad der eingesetzten Diurethane, und so ist es nicht verwunderlich, daß bei all den bekannten Verfahren zur phosgenfreien Urethanherstellung ein großer Aufwand für die Reinigung der Diurethane aufgebracht werden muß.

Aus EP-A-0 547 444 ist ein Verfahren zur Herstellung von hochreinen aromatischen Diund/oder Polyurethanen bekannt, bei dem das nach der Umsetzung vorliegende Reaktionsgemisch von gegebenenfalls verwendetem Lösungsmittel befreit und die gebildeten aromatischen Di- und/oder Polyurethane durch Extraktion mit Wasser gereinigt werden.

Die destillative Reinigung von Diurethanen ist bislang technisch noch nicht durchgeführt worden, da Diurethane thermolabile Verbindungen sind, die nur unter Inkaufnahme von Ausbeuteverlusten zu destillieren sind. Selbst bei der Destillation der im Vergleich zu den aromatischen Diurethanen stabileren aliphatischen oder cycloaliphatischen Diurethane fallen nicht unerhebliche Mengen an Zersetzungsprodukten im Destillationssumpf an.

Es war daher äußerst überraschend, daß Diurethane destilliert werden konnten, wenn die Destillation in Gegenwart eines Trägergases durchgeführt wurde, wobei als Trägergas ein niedrigsiedender Alkohol verwendet wurde. Diese Tatsache ist insofern überraschend, da bei Verwendung eines anderen Trägergases, wie z.B. Stickstoff, Toluol oder Wasser, schlechtere Destillationsergebnisse erzielt wurden.

Gegenstand der vorliegenden Erfindung ist somit ein Verfahren zur destillativen Reinigung von Diurethanen der Formel (I)

R¹ - (NHCOOR²)₂ (I),

wobei
- R¹: für einen gegebenenfalls substituierten aromatischen, araliphatischen, cycloaliphatischen oder aliphatischen Kohlenwasserstoffrest mit 4 bis 10 Kohlenstoffatomen und
- R²: für einen Alkylrest mit 1 bis 6 Kohlenstoffatomen oder einen cycloaliphatischen Kohlenwasserstoffrest mit 5 bis 6 Kohlenstoffatomen
stehen
und als Substituenten für den Kohlenwasserstoffrest R¹ Alkylgruppen mit 1 bis 4 Kohlenstoffatomen vorgesehen sind,
das dadurch gekennzeichnet ist, daß die Destillation der Diurethane in Gegenwart von niedrigsiedenden Alkoholen der Formel (II)

R³OH (II),

wobei
- R³: die Bedeutung von R² hat,
durchgeführt wird, wobei die Diurethane zusammen mit dem Alkoholträgerdampf als Destillat anfallen.
Bevorzugte Beispiele der aromatischen Diurethane sind O-Methyl-, O-Ethyl-, O-Propyl-, O-Isopropyl-, O-Butyl-, O-Hexyl-, O-Neopentyl- und O-Cyclohexylurethane auf Basis verschiedener Diamine, wie z.B. m-Phenylen-, p-Phenylen- und 2,4-Toluylendiamin, sowie Gemische aus 2,4-Toluylendiamin und 2,6-Toluylendiamin.

Als Beispiel der bevorzugten Diurethane seien genannt: N,N'-m-Phenylen-, N,N'p-Phenylen-bis(O-butylurethan) und deren Gemische sowie N,N'-2,4-Toluylen-bis-(O-butylurethan) und dessen Gemische mit N,N'-2,6-Toluylen-bis(O-butylurethan).

Bevorzugte Beispiele der araliphatischen Diurethane sind O-Methyl-, O-Ethyl-, O-Propyl-, O-Isopropyl-, O-Butyl-, O-Hexyl-, O-Neopentyl- und O-Cyclohexylurethane auf Basis verschiedener Diamine, wie m-, p-Xylylendiamin oder deren Gemische, wobei O-Butylurethane besonders bevorzugt sind.

Beispiele für cycloaliphatische Diurethane sind O-Methyl-, O-Ethyl-, O-Propyl-, O-Isopropyl-, O-Butyl-, O-Hexyl-, O-Neopentyl- und O-Cyclohexylurethane auf Basis verschiedener Diamine, wie Isophorondiamin und 1-Amino-1-methyl-4(3)-aminomethyl-cyclohexan.

Beispiele für aliphatische Diurethane sind O-Methyl-, O-Ethyl-, O-Propyl-, O-Isopropyl-, O-Butyl-, O-Hexyl-, O-Neopentyl- und O-Cyclohexylurethane auf Basis verschiedener Diamine, wie Butylen- und Hexamethylendiamin.

Als typische Beispiele für Diurethane der Formel (I) seien genannt: N,N'-m- und N,N'-p-Phenylen-bis(O-butylurethan), N,N'-2,4(6)-Toluylen-bis(Obutylurethan), N,N'-m- und N,N'-p-Xylylen-bis(O-butylurethan), N,N'-Isophoronbis(O-butylurethan), 1-Butoxycarbonylamino-1-methyl-4(3)-butoxycarbonylaminocyclohexan, N,N'-Hexamethylen-bis(O-butylurethan) sowie N,N'-Butylenbis(O-butylurethan), insbesondere N,N'-2,4(6)-Toluylen-bis(O-butylurethan), N,N'-Hexamethylen-bis(O-butylurethan), N,N'-Isophoron-bis(O-butylurethan) und deren Gemische.

Die erfindungsgemäße Reinigung von Diurethanen der Formel (I) erfolgt durch Trägergasdestillation, wobei als niedrigsiedende Alkohole (Siedepunktbereich von 60 bis 160°C), insbesondere solche Alkohole, die den Diurethanen zugrunde liegen, als Trägergas verwendet werden. In diesem bevorzugten Fall ist also der Alkylrest R³ in Formel (II) identisch mit dem Alkylrest R² der Formel (I). Als Beispiele für die als Trägergas geeigneten Alkohole seien genannt: Methanol, Ethanol, Propanol, Isopropanol, Butanol, Neopentylalkohol, Hexanol und/oder Cyclohexanol. Andere üblicherweise bei der Trägergasdestillation als Trägergas verwendete Stoffe, wie z.B. Kohlendioxid, Stickstoff und/oder Wasserdampf oder deren Gemische, sind für die erfindungsgemäße Reinigung der Diurethane weniger geeignet, da diese eine geringere Destillationsausbeute bewirken. Es ist jedoch möglich, die niedrigsiedenden Alkohole im Gemisch mit den üblichen, bei der Trägergasdestillation verwendete Stoffe bei der Destillation einzusetzen. Zweckmäßigerweise wird der den Diurethanen zugrundeliegende Alkohol als Trägergas verwendet. Selbstverständlich ist es aber auch möglich, andere Alkohole der Formel (II) als Trägergas einzusetzen.

Die erfindungsgemäßen Diurethane werden auf phosgenfreiem Wege gemäß dem Stand der Technik hergestellt, wobei gegebenenfalls zuerst die als Rohprodukt anfallenden Diurethane gegebenenfalls durch Destillation von niedrigsiedenden Bestandteilen wie Lösungsmitteln oder niedrigsiedenden Ausgangs- und/oder Nebenprodukten oder sonstigen niedersiedenden Hilfsstoffen befreit werden. Dieser gegebenenfalls als Destillation durchgeführte Reinigungsschritt kann unter Normaldruck oder unter vermindertem Druck durchgeführt werden. Nach Abtrennung der niedrigsiedenden Komponenten wird das Produktgemisch, das zu über 60 Gew.-% aus Diurethanen der Formel (I) besteht, gemäß dem erfindungsgemäßen Verfahren gereinigt. Der Rest (<40 Gew.-%) besteht aus höhersiedenden Nebenprodukten und/oder anderen Verunreinigungen, wie Aminourethanen, Harnstoffurethanen, Oligo- oder Polyharnstoffen und Zersetzungsprodukten von Harnstoff oder anderen Kohlensäurederivaten.

Die Trägergasdestillation zur Reinigung von Diurethanen der Formel (I) erfolgt im Temperaturbereich von 100 bis 350°C, vorzugsweise jedoch bei Temperaturen von 150 bis 250°C. Da die erfindungsgemäßen Diurethane temperaturempfindlich sind, wird die Trägergasdestillation bei möglichst niedrigen Drücken unter Vakuum im Bereich von 0,01 bis 200 mbar, vorzugsweise bei 0,1 bis 50 mbar, durchgeführt.

Das erfindungsgemäße Verfahren wird so durchgeführt, daß das Gewichtsverhältnis von Alkohol der Formel (II) zu Diurethan der Formel (I) 0,01 bis 500, vorzugsweise jedoch 0,5 bis 10 beträgt.

Die Trägergasdestillation kann diskontinuierlich oder kontinuierlich, ein- oder mehrstufig, gegebenenfalls unter Mitverwendung von trennwirksamen Kolonnen, z.B. Packungskolonnen, im Gleich- oder im Gegenstrom oder unter Verwendung von Verdampfern, z.B. Dünnschicht- oder Fallfilmverdampfern, durchgeführt werden.

Nach einer möglichen Variante des erfindungsgemäßen Verfahrens werden die von niedrigsiedenden Komponenten befreiten Diurethane in der Destillationsblase vorgelegt. Der als Trägergas verwendete Alkohol der Formel (II) wird aus einem geeigneten Behälter mittels einer geeigneten Förderpumpe über eine beheizbare Kapillare gasförmig in den Sumpfkolben dosiert oder direkt als gegebenenfalls überhitzter Dampf in den Sumpfkolben geführt. Außerdem ist es möglich, den Alkohol flüssig in den Sumpfkolben zu fördern und während der Destillation zu verdampfen. Der Kühler der Destillationsapparatur und die Kühlleistung werden so gewählt, daß zumindest das erfindungsgemäße Diurethan vollständig, gegebenenfalls zusammen mit dem als Trägergas verwendeten Alkohol kondensiert und in der Destillationsvorlage aufgefangen wird. Falls eine vollständige Kondensation des Trägergases angestrebt wird, kann auch der Alkohol der Formel (II) in der Destillationsvorlage vorgelegt werden. Die Destillation wird unter Vakuum durchgeführt. Durch eine ausreichende Beheizung wird dafür gesorgt, daß das erfindungsgemäße Diurethan stets in Gegenwart von dampfförmigem Alkohol überdestilliert.

Nach einer weiteren, energetisch vorteilhaften Variante des erfindungsgemäßen Verfahrens werden die von den niedrigsiedenden Komponenten befreiten Diurethane bei reduziertem Druck auf die Packung oder Füllkörperschüttung einer Destillationskolonne aufgegeben. Im Gegenstrom zum Diurethan strömt der als Trägergas der Formel (II) eingesetzte Alkohol von unten nach oben durch die Packung und trägt das Diurethan über Kopf aus. Die Verdampfungswärme für das Diurethan liefert das Trägergas, welches abkühlt. Zwischen Kolonnenpackungsoder Füllkörperschüttungssegmenten kann das Trägergas bei Bedarf zwischenerhitzt werden.

Das über Kopf abströmende Trägergas-Diurethan-Gasgemisch wird in einem folgenden Kondensator soweit kondensiert, daß das Diurethan nahezu vollständig kondensiert und der als Trägergas verwendete Alkohol gasförmig bleibt. Bei der Kondensation evtl. auftretender Diurethan-Nebel wird in einem folgenden Abscheider abgetrennt.

Das von Diurethan befreite Trägergas wird von einem Gasverdichter angesaugt und, nachdem es in einem Wärmetauscher auf die erforderliche Temperatur aufgeheizt worden ist, in die Destillationskolonne gefördert.

Die nach Durchführung des erfindungsgemäßen Verfahrens gereinigten Diurethane der Formel (I) werden gegebenenfalls von dem als Trägergas verwendeten Alkohol abgetrennt. Diese Trennung kann durch Kristallisation oder Extraktion, vorzugsweise allerdings durch Destillation erfolgen, wobei die Destillation gegebenenfalls unter vermindertem Druck durchgeführt wird. Die dabei anfallenden Diurethane können in einer geeigneten Spaltapparatur thermisch (150 bis 350°C; Druck vorzugsweise 0,01 mbar bis 1 bar) in Diisocyanate und die zugrundeliegenden Alkohole der Formel R²OH gespalten werden und sind somit zur phosgenfreien Herstellung von Diisocyanaten geeignet.

Anhand der nachfolgend angeführten Beispiele soll die Erfindung näher erläutert werden. Alle Prozentangaben beziehen sich, falls nicht anders erwähnt, auf Gewichtsprozent.

### Beispiele

### Beispiel 1

In der Sumpfblase einer Destillationsapparatur ohne Kolonne werden 113,0 g N,N'-2,4-Toluylen-bis(O-butylurethan) vorgelegt und unter Vakuum (0,5 bis 0,6 mbar) und Rühren auf 180°C erhitzt. Dann wird mit Hilfe einer Telabpumpe über eine Kapillare Butanol in den Sumpfkolben dosiert und die Sumpftemperatur langsam bis 220°C erhöht, so daß Butanol und N,N'-2,4-Toluylen-bis(O-butylurethan) miteinander destillieren. Die Dämpfe werden mit Hilfe eines mit Wasser betriebenen Liebigkühlers kondensiert und in einer mit Aceton/Trockeneis gekühlten Vorlage aufgefangen. Während der Destillation werden 135 ml (109,3 g) Butanol in den Sumpfkolben eindosiert. Der Volumenstrom an Butanol beträgt ca. 3 ml/min.

Der Vorlagekolben wird am Rotationsverdampfer bis zur Trockne eingeengt. Dabei fallen 109,6 g N,N'-2,4-Toluylen-bis(O-butylurethan) mit einer Reinheit 96,1 Gew.-% (HPLC-Analyse) an. Im Sumpfkolben verbleiben 0,6 g Feststoff, die Restmenge befindet sich in den Kühlfallen.

### Vergleichsbeispiel 1 (ohne Trägergas)

In der oben beschriebenen Destillationsapparatur werden 115 g N,N'-2,4-Toluylenbis(O-butylurethan) gleicher Provenienz ohne Trägergas unter Vakuum (0,4 bis 0,6 mbar) destilliert, indem die Sumpftemperatur langsam bis 220°C angezogen wird. Da bei dieser Temperatur noch erhebliche Mengen an Einsatzprodukt im Sumpfkolben verbleiben und die Destillation nachläßt, wird die Sumpftemperatur langsam auf 250°C gesteigert. Dabei finden bereits Zersetzungsreaktionen statt, das Diurethan beginnt zu gasen, so daß das Vakuum von 0,6 auf 0,8 mbar fällt.

Im Vorlagekolben werden lediglich 82,7 g eines Feststoffes gewonnen, der nur zu 54,0 Gew.-% (HPLC-Analyse) aus N,N'-2,4-Toluylen-bis(O-butylurethan) besteht. Als Destillationsrückstand fallen 6,5 g eines bei 254°C unter Zersetzung schmelzenden Feststoffes an. Die Restmenge befindet sich in den Kühlfallen.

### Vergleichsbeispiel 2 (Trägergas: Toluol)

Wie in Beispiel 1 beschrieben, werden 113,0 g N,N'-2,4-Toluylen-bis(O-butylurethan) destilliert. Während der Destillation werden 115 ml (100,2 g) Toluol als Trägergas zudosiert. Der Vorlagekolben wird am Rotationsverdampfer bis zur Trockene eingeengt. Dabei fallen 102,8 g Feststoff an, der laut HPLC-Analyse zu 84,6 Gew.-% aus N,N'-2,4-Toluylen-bis(O-butylurethan) besteht. Als Destillationsrückstand fallen 5,8 g eines Feststoffs an, der bei Temperaturen bis 250°C nicht vollständig geschmolzen werden kann.

### Vergleichsbeispiel 3 (Trägergas: Wasser)

Wie in Beispiel 1 beschrieben, werden 113,0 g N,N'-2,4-Toluylen-bis(O-butylurethan) destilliert.

Während der Destillation werden 100 g Wasser als Trägergas zudosiert. Der Vorlagekolben wird am Rotationsverdampfer bis zur Trockene eingeengt. Dabei fallen 37,5 g Feststoff an, der laut HPLC-Analyse zu 82,5 Gew.-% aus N,N'-2,4-Toluylen-bis(O-butylurethan) besteht. Der Destillationsrückstand im Sumpfkolben beträgt 55,5 g.

### Vergleichsbeispiel 4 (Trägergas: Stickstoff)

Wie in Beispiel 1 beschrieben, werden 113 g N,N'-2,4-Toluylen-bis(O-butylurethan) unter Vakuum auf 180°C erhitzt und über eine Kapillare 83 ml/min Stickstoff eingeleitet. Die Destillation findet bei einer Sumpftemperatur von 180 bis 220°C statt. Da bei diesen Temperaturen noch erhebliche Mengen im Sumpfkolben verbleiben und die Destillation nachläßt, wird die Sumpftemperatur auf 240°C erhöht. Das Destillat (102,8 g) wird in einer mit Aceton/Trockeneis gekühlten Vorlage gesammelt. Es besteht laut HPLC-Analyse zu 93,9 Gew.-% aus N,N'-2,4-Toluylen-bis(O-butylurethan). Im Sumpfkolben verbleiben 8,7 g Feststoff, der im Temperaturbereich von 147 bis 158°C unter Zersetzung schmilzt.

### Beispiel 2

Im Sumpfkolben der oben beschriebenen Destillationsapparatur werden 100 g N,N'-2,4-Toluylen-bis(O-ethylurethan), in der Destillationsvorlage vorgelegt und unter Rühren und Vakuum (2,5 bis 3,0 mbar) auf 150°C erwärmt. Danach wird über eine Telabpumpe Ethanol eindosiert und bei einer Sumpftemperatur von 150 bis 238°C destilliert. Während der Destillation werden 29 g Ethanol zudosiert. Das aus Ethanol und N,N'-2,4-Toluylen-bis(O-ethylurethan) bestehende Destillat wird in einer mit Aceton/Trockeneis gekühlten Vorlage gesammelt, Ethanol am Rotationsverdampfer abgezogen. Der zurückbleibende Feststoff (83,0 g) besteht laut HPLC-Analyse zu 96,0 Gew.-% aus N,N'-2,4-Toluylen-bis(O-ethylurethan).

## Patentansprüche

1. Verfahren zur destillativen Reinigung von Diurethanen der Formel (I)
R¹ - (NHCOOR²)₂ (I),
wobei
R¹ für einen gegebenenfalls substituierten aromatischen, araliphatischen, cycloaliphatischen oder aliphatischen Kohlenwasserstoffrest mit 4 bis 10 Kohlenstoffatomen und
R² für einen Alkylrest mit 1 bis 6 Kohlenstoffatomen oder einen cycloaliphatischen Kohlenwasserstoffrest mit 5 bis 6 Kohlenstoffatomen
stehen
und als Substituenten für den Kohlenwasserstoffrest R¹ Alkylgruppen mit 1 bis 4 Kohlenstoffatomen vorgesehen sind,
**dadurch gekennzeichnet, daß** die Destillation der Diurethane in Gegenwart von niedrigsiedenden Alkoholen der Formel (II)
R³OH (II)
wobei
R³ die Bedeutung von R² hat,
durchgeführt wird, wobei die Diurethane zusammen mit dem Alkoholträgerdampf als Destillat anfallen.

2. Verfahren gemäß Anspruch 1, **dadurch gekennzeichnet, daß** es sich bei den Diurethanen der Formel (I) um O-Methyl-, O-Ethyl-, O-Propyl-, O-Isopropyl-, O-Butyl-, O-Neopentyl-, O-Hexyl- und O-Cyclohexylurethane auf Basis von m- und p-Phenylendiamin, 2,4(6)-Toluylendiamin, m- oder p-Xylylendiamin, Isophorondiamin, 1-Amino-1-methyl-4(3)aminomethylcyclohexan, 1,4-Diaminobutan, Hexamethylendiamin oder Gemische dieser Diurethane handelt.

3. Verfahren gemäß Anspruch 1, **dadurch gekennzeichnet, daß** es sich bei den niedrigsiedenden Alkoholen der Formel (II) um solche Alkohole handelt, die den zur destillativen Reinigung eingesetzten Diurethanen zugrundeliegen.

4. Verfahren gemäß Anspruch 1, **dadurch gekennzeichnet, daß** es sich bei den Diurethanen der Formel (I) um die bei der phosgenfreien Herstellung von Diurethanen anfallenden Produkte handelt, die gegebenenfalls durch Destillation von Lösungsmitteln, niedrigsiedenden Ausgangs- und/oder niedrigsiedenden Nebenprodukten befreit sind und deren Anteil an höhersiedenden Nebenprodukten und/oder anderen Verunreinigungen bis zu 40 Gew.-% beträgt.

5. Verfahren gemäß Ansprüchen 1 bis 4, **dadurch gekennzeichnet, daß** die Trägerdampfdestillation bei Temperaturen von 100 bis 350°C und Drücken von 0,01 bis 200 mbar durchgeführt wird.

6. Verfahren gemäß Ansprüchen 1 bis 5, **dadurch gekennzeichnet, daß** das Gewichtsverhältnis von Alkohol der Formel (II) zu Diurethan der Formel (I) 0,01 bis 500 beträgt.

## Claims

1. Process for the distillative purification of diurethanes of the formula (I)
R¹ - (NHCOOR²)₂ (I),
where
R1 denotes an optionally substituted aromatic, araliphatic, cycloaliphatic or aliphatic hydrocarbon radical with 4 to 10 carbon atoms, and
R2 denotes an alkyl radical with 1 to 6 carbon atoms or a cycloaliphatic hydrocarbon radical with 5 or 6 carbon atoms,
wherein
the substituents in the hydrocarbon radical R1 are alkyl groups with 1 to 4 carbon atoms,
**characterised in that** the distillation of the diurethanes is carried out in the presence of low-boiling alcohols of the formula (II)
R³OH (II),
where
R3 denotes the same as R2,
the diurethanes together with the alcohol carrier vapour occuring as distillate.

2. Process according to claim 1, **characterised in that** the diurethanes of the formula (I) are O-methyl, O-ethyl, O-propyl, O-isopropyl, O-butyl, O-neopentyl, O-hexyl and O-cyclohexyl urethane based on m-phenylenediamine and p-phenylenediamine, 2,4(6)-toluylenediamine, m-xylylenediamine or p-xylylenediamine, isophorone diamine, 1-amino-1-methyl-4(3)aminomethyl cyclohexane, 1,4-diaminobutane, hexamethylenediamine or mixtures of these diurethanes.

3. Process according to claim 1, **characterised in that** the low-boiling alcohols of the formula (II) are alcohols on which are based the diurethanes used for the distillative purification.

4. Process according to claim 1, **characterised in that** the diurethanes of the formula (I) are the products formed in the phosgene-free preparation of diurethanes, which are optionally freed by distillation from solvents, low-boiling starting materials and/or low-boiling secondary products, and whose proportion of higher-boiling secondary products and/or other impurities is up to 40 wt.%.

5. Process according to claims 1 to 4, **characterised in that** the carrier gas distillation is carried out at temperatures of 100°C to 350°C and at pressures of 0.01 to 200 mbar.

6. Process according to claims 1 to 5, **characterised in that** the weight ratio of alcohol of the formula (II) to diurethane of the formula (I) is 0.01 to 500.

## Revendications

1. Procédé pour la purification par distillation de diuréthannes de la formule (I)
R¹ - (NHCOOR²)₂ (I),
dans laquelle
R¹ représente un reste hydrocarboné aromatique, araliphatique, cycloaliphatique ou aliphatique éventuellement substitué avec de 4 à 10 atomes de carbone, et
R² représente un reste alkyle avec de 1 à 6 atomes de carbone ou un reste hydrocarboné cycloaliphatique avec de 5 à 6 atomes de carbone
et sont prévus comme substituants pour le reste hydrocarboné R¹ des groupes alkyle avec de 1 à 4 atomes de carbone,
**caractérisé en ce que** l'on réalise la distillation des diuréthannes en présence d'alcools de faible point d'ébullition de la formule (II)
R³OH (II)
dans laquelle
R³ a la signification de R² ;
les diuréthannes étant produits comme distillat avec les vapeurs de l'alcool porteur.

2. Procédé selon la revendication 1, **caractérisé en ce qu'**il s'agit pour les diuréthannes de la formule (I) de l'o-méthyl-, l'o-éthyl-, l'o-propyl-, l'o-isopropyl-, l'o-butyl-, l'o-néopentyl-, l'o-hexyl-, et l'o-cyclohexyluréthanne à base de m- et de p-phénylènediamine, de 2,4(6)-toluylènediamine, de m- ou de p-xylylènediamine, d'isophoronediamine, de 1-amino-1-méthyl-4(3)aminométhylcyclohexane, de 1,4-diaminobutane, d'hexaméthylènediamine ou de mélanges de ces diuréthannes.

3. Procédé selon la revendication 1, **caractérisé en ce qu'**il s'agit pour les alcools de faible point d'ébullition de la formule (II) d'alcools qui sont à la base des diuréthannes utilisés pour une purification par distillation.

4. Procédé selon la revendication 1, **caractérisé en ce qu'**il s'agit pour les diuréthannes de la formule (I) des produits obtenus lors de la préparation sans phosgène de diuréthannes, lesquels sont éventuellement libérés par distillation de solvants, de produits de départ de faible point d'ébullition et/ou de produits secondaires de faible point d'ébullition et dont la part en produits secondaires et/ou en d'autres impuretés de point d'ébullition plus élevé est d'au plus 40 % en poids.

5. Procédé selon les revendications 1 à 4, **caractérisé en ce que** la distillation avec un gaz porteur est réalisée à des températures de 100 à 350°C et à des pressions de 0,01 à 200 mbar.

6. Procédé selon les revendications 1 à 5, **caractérisé en ce que** le rapport massique de l'alcool de la formule (II) au diuréthanne de la formule (I) est compris entre 0,01 et 500.
